(19)

Europäisches
Patentamt
European
Patent Office
Office européen
des brevets

(11)  **EP 4 545 572 A1**

(12)  **EUROPEAN PATENT APPLICATION**
published in accordance with Art. 153(4) EPC

(43) Date of publication:
**30.04.2025 Bulletin 2025/18**

(21) Application number: **23827062.3**

(22) Date of filing: **13.06.2023**

(51) International Patent Classification (IPC):
*C08B 37/04* (2006.01)      *A61K 31/734* (2006.01)
*A61L 31/04* (2006.01)      *A61P 7/04* (2006.01)

(52) Cooperative Patent Classification (CPC):
**A61K 31/734; A61L 31/04; A61P 7/04;
C08B 37/0084**

(86) International application number:
**PCT/JP2023/021937**

(87) International publication number:
**WO 2023/248876 (28.12.2023 Gazette 2023/52)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **24.06.2022 JP 2022101906**

(71) Applicants:
• **OSAKA UNIVERSITY
Suita-shi
Osaka 565-0871 (JP)**
• **Kaigen Pharma Co., Ltd.
Osaka-shi, Osaka 541-0045 (JP)**

(72) Inventors:
• **NAKAJIMA, Kiyokazu
Suita-shi, Osaka 565-0871 (JP)**

• **SUZUKI, Sayaka
Otaru-shi, Hokkaido 047-0013 (JP)**
• **SATO, Masahiro
Otaru-shi, Hokkaido 047-0013 (JP)**
• **NISHIDA, Ryuichi
Kawachinagano-shi, Osaka 586-0006 (JP)**
• **YAMAUCHI, Kosuke
Kawachinagano-shi, Osaka 586-0006 (JP)**
• **AZUMA, Kento
Kawachinagano-shi, Osaka 586-0006 (JP)**
• **YAMAMOTO, Junji
Kawachinagano-shi, Osaka 586-0006 (JP)**
• **YOSHIHARA, Takahiro
Kawachinagano-shi, Osaka 586-0006 (JP)**
• **YAMADA, Katsuki
Hirakata-shi, Osaka 573-1132 (JP)**

(74) Representative: **Cabinet Beau de Loménie
158, rue de l'Université
75340 Paris Cedex 07 (FR)**

(54)  **ALGINIC ACID SALT AND HEMOSTATIC MATERIAL CONTAINING SAME**

(57)    The present invention aims to provide an alginate with a higher hemostatic effect than conventional alginates. The alginate of the present invention contains a calcium salt of a carboxyl group and a different carboxyl (salt) group, wherein, provided that a structural unit containing the calcium salt in a repeating unit (structural unit) constituting the alginate is calcium alginate, a structural unit containing the different carboxyl (salt) group in the repeating unit is sodium alginate, and the alginate is regarded as a mixture of the sodium alginate and the calcium alginate, the alginate has a mass percentage of the calcium alginate (calcium content) of 1 to 99% by mass.

EP 4 545 572 A1

Processed by Luminess, 75001 PARIS (FR)

## Description

TECHNICAL FIELD

The present invention relates to alginates and hemostatic materials containing the alginates.

BACKGROUND ART

Alginic acid is a polysaccharide contained in brown algae. It is a polymer of mannuronic acid and/or gluronic acid.

Alginic acid is insoluble in water. In contrast, alginates such as sodium alginate and potassium alginate are easily dissolved in water to exhibit a thickening effect. Alginates easily form gel when they coexist with calcium ions. Thus, alginates are widely used as a thickener, a gelling agent, a stabilizer, or the like in the fields of food, pharmaceuticals, medical materials, cosmetics, etc.

Alginic acid and alginates have a nature of forming gel upon reaction with water. Taking the advantage of this nature, they have been examined as hemostatic medical materials, etc.

For example, Patent Literature 1 discloses a solid pharmaceutical composition containing (A) a gelling agent, (B) a salt of a divalent metal and at least one acid selected from the group consisting of organic acids and inorganic acids, and (C) polyethylene glycol.

Patent Literature 2 discloses a kit for producing an alginate gel including: a first container containing soluble alginate and a second container containing insoluble alginate/gelling ion particles.

CITATION LIST

- Patent Literature

    Patent Literature 1: WO 2013/077414
    Patent Literature 2: JP 2008-515927 T

SUMMARY OF INVENTION

- Technical Problem

As described above, alginates have been used in various applications, including hemostatic materials. Still, there is room for improvement to further increase the hemostatic effect.

The present invention was made in view of the current situation described above and aims to provide an alginate with a higher hemostatic effect than conventional alginates.

- Solution to Problem

As a result of various studies on alginates, the present inventors have found that an alginate containing a calcium salt of a carboxyl group and having a calcium content of a carboxyl (salt) group within a specific range exhibits a high hemostatic effect. The alginate can perfectly solve the problem. Accordingly, the present invention was completed.

The present invention encompasses the alginate, etc. described below.

    [1] An alginate, containing

        a calcium salt of a carboxyl group and
        a different carboxyl (salt) group,
        wherein, provided that a structural unit containing the calcium salt in a repeating unit (structural unit) constituting the alginate is calcium alginate, a structural unit containing the different carboxyl (salt) group in the repeating unit is sodium alginate, and the alginate is regarded as a mixture of the sodium alginate and the calcium alginate, the alginate has a mass percentage of the calcium alginate (calcium content) of 1 to 99% by mass.

    [2] The alginate according to the above [1],
    wherein the alginate has a mass average molecular weight of 10000 or more.
    [3] The alginate according to the above [1] or [2],

2

wherein the alginate contains a mannuronate-derived structural unit and a gluronate-derived structural unit, and when all cations in the alginate are replaced by protons, the alginate has a ratio of a mannuronic acid-derived structural unit to a gluronic acid-derived structural unit (mannuronic acid/gluronic acid) of 0.6 or more.

[4] The alginate according to any one of the above [1] to [3],
wherein the alginate has a bulk density of 0.2 $g/cm^3$ or more.
[5] A hemostatic material containing the alginate according to any one of the above [1] to [4].

- Advantageous Effects of Invention

The alginate of the present invention having the above-described structure exhibits an excellent hemostatic effect and therefore can suitably be used as a hemostatic material, etc.

BRIEF DESCRIPTION OF DRAWINGS

FIG. 1 includes diagrams schematically showing injured areas of Grades A to D according to the "Evaluation criteria for the degree of hemostasis" in Table 2.
FIG. 2 is a photograph showing a microscopic observation result of a cross-section of an area where alginate 9 obtained in Example 9 was applied to a bleeding site and then hemostasis was confirmed five minutes later.

DESCRIPTION OF EMBODIMENTS

A preferred embodiment of the present invention is specifically described below. Yet, the present invention is not limited to the following description, and modification may suitably be made without departing from the gist of the present invention. A combination of two or more preferred embodiments of the present invention described below also corresponds to a preferred embodiment of the present invention.

<Alginate>

The alginate of the present invention contains a calcium salt of a carboxyl group. In its molecule, the calcium salt of a carboxyl group is present together with a different carboxyl (salt) group such as a sodium salt of a carboxyl group.
For example, Patent Literature 1 discloses a mixture of sodium alginate and a calcium salt, while Patent Literature 2 discloses a mixture of sodium alginate and calcium alginate. In a molecule of such mixtures, a calcium salt of a carboxyl group and a different carboxyl (salt) group are not present together. Therefore, the alginate of the present invention differs from the mixtures.
Presumably, the alginate of the present invention rapidly comes in contact with moisture in blood to embolize the bleeding site, thereby exhibiting a local hemostatic effect.
The alginate of the present invention contains a repeating unit including a mannuronic acid (salt)-derived structural unit and/or a gluronic acid (salt)-derived structural unit. The alginate includes a calcium salt group-containing structural unit that is a structural unit derived from a calcium salt of mannuronic acid or gluronic acid, and a different carboxyl (salt) group-containing structural unit that is a structural unit not derived from mannuronic acid, gluronic acid, or calcium salts of these acids.
According to the alginate of the present invention, provided that a structural unit containing the calcium salt in a repeating unit (structural unit) constituting the alginate is calcium alginate, a structural unit containing the different carboxyl (salt) group in the repeating unit is sodium alginate, and the alginate is regarded as a mixture of the sodium alginate and the calcium alginate, the alginate has a mass percentage of the calcium alginate (calcium content) of 1 to 99% by mass.
In a molecule of the alginate of the present invention, the calcium salt of a carboxyl group is present together with the different carboxyl (salt) group; in other words, a structural unit containing the calcium salt is present together with a structural unit containing the different carboxyl (salt) group. The calcium content is calculated while the structural unit containing the calcium salt is regarded as calcium alginate having the same number of the structural units and a structural unit containing the different carboxyl (salt) group is regarded as sodium alginate having the same number of the structural units so that the alginate is regarded as a mixture of the sodium alginate and the calcium alginate.
The hemostatic effect in the present invention is due to the ion strength of calcium ions contained in a specific ratio in the alginate. Thus, the kinds of cations other than calcium in the alginate are not limited as long as the calcium content is within the above range.
The calcium content can be measured by X-ray fluorescence analysis as described in EXAMPLES.

For example, in the case where the alginate is a potassium/calcium alginate, potassium alginate instead of sodium alginate is used as a reference material in the fluorescent X-ray measurement, and then the intensities of the fluorescent X-rays of the potassium element are measured. Yet, the mass percentage is calculated on the assumption that the potassium in the alginate is sodium.

Specifically, mass percentages of calcium alginate and potassium alginate are calculated based on the intensities of fluorescent X-rays of the calcium element and the potassium element. Then, the mass percentage of the potassium alginate is converted to a chemically equivalent mass percentage of potassium. This value is multiplied with the molar mass of sodium alginate ($NaC_6H_7O_6$). The percentage of the calcium alginate based on a sum of the multiplied value and the mass percentage of the calcium alginate taken as 100 is determined as a mass percentage of the calcium alginate.

In the case of an alginate containing an acidic carboxyl group, an acidic alginic acid instead of sodium alginate is used as a reference material in the fluorescent X-ray measurement. Yet, the mass percentage is calculated on the assumption that the proton in alginate is sodium.

The calcium content of the alginate is preferably 10 to 95% by mass. The alginate with a calcium content within the range exhibits a higher hemostatic effect.

The calcium content is more preferably 20 to 90% by mass, still more preferably 25 to 85% by mass, further preferably 30 to 80% by mass, further more preferably 40 to 80% by mass, particularly preferably 50 to 80% by mass.

As long as the calcium content of the alginate is within the above range, the cation of a carboxyl (salt) group other than the calcium salt group is not limited. Examples of the cation include sodium ion, potassium ion, ammonium ion, and proton. The alginate may contain two or more kinds of monovalent cations. Of these, sodium ion and potassium ion are preferred, with sodium ion being more preferred.

An embodiment of the alginate in which the carboxyl (salt) group is a calcium salt group or a sodium salt group, i.e., sodium/calcium alginate is one of preferred embodiments of the present invention.

The mass average molecular weight of the alginate is not limited but is preferably 10000 or more. The alginate having such a mass average molecular weight exhibits a higher hemostatic effect. The mass average molecular weight is more preferably 10000 to 10000000, still more preferably 20000 to 5000000, further preferably 30000 to 3000000, further more preferably 40000 to 2000000, still further more preferably 50000 to 1600000, particularly preferably 100000 to 1000000, more particularly preferably 200000 to 800000, most preferably 300000 to 600000.

In one embodiment, the mass average molecular weight of the alginate may be 500000 or less.

The mass average molecular weight can be measured by the method described in EXAMPLES.

The alginate contains a mannuronate-derived structural unit and/or a gluronate-derived structural unit. It may be a polymer consisting only of a mannuronate-derived structural unit or may be a polymer consisting only of a gluronate-derived structural unit. Preferably, the alginate is a copolymer containing a mannuronate-derived structural unit and a gluronate-derived structural unit.

Herein, the mannuronate-derived structural unit refers to a structural unit obtained by replacing at least one of hydroxy groups in a mannuronate with an ether group. Likewise, the gluronate-derived structural unit refers to a structural unit obtained by replacing at least one of hydroxy groups in a gluronate with an ether group.

In the case where the alginate is a copolymer containing a mannuronate-derived structural unit and a gluronate-derived structural unit, the ratio between the mannuronate-derived structural unit and the gluronate-derived structural unit is not limited. When all cations in the alginate are replaced by protons, in other words, when the alginate is regarded as alginic acid, the alginate preferably has a ratio of the mannuronic acid-derived structural unit to the gluronic acid-derived structural unit (mannuronic acid/gluronic acid, hereinafter, also referred to as M/G ratio) of 0.6 or more. The alginate having such a M/G ratio exhibits a higher hemostatic effect. The M/G ratio is more preferably 0.6 to 5, still more preferably 0.67 to 4, further preferably 0.7 to 4, further more preferably 0.8 to 3, still further more preferably 0.9 to 2, particularly preferably 0.9 to 1.5.

The ratio between the mannuronic acid-derived structural unit and the gluronic acid-derived structural unit can be measured as described below in accordance with the method in Carbohydrate Research, 32(1974), 217-225.

Alginic acid is a complex block copolymer consisting of blocks bonded in any order: a MM block consisting only of a mannuronic acid-derived structural unit; a GG block consisting only of a gluronic acid-derived structural unit; and a MG block consisting of a mannuronic acid-derived structural unit and a gluronic acid-derived structural unit. Alginic acid has a different resistance to hydrolysis and a different solubility in an acid depending on the volumetric ratio and the array of these blocks.

Based on the nature of alginic acid, alginic acid is degraded with weak hydrochloric acid under conditions to be cut into a MM block, a GG block, and a MG block, and then the blocks are fractioned by a usual method. The saccharide content of each block is measured by the phenol-sulfuric acid method, and then a M/G ratio is calculated by the following equation.

M/G = (saccharide content of MM block + (saccharide content of MG block/2))/(saccharide content of GG block + (saccharide content of MG block/2))

The alginate preferably has a bulk density of 0.2 g/cm$^3$ or more. The alginate having such a bulk density exhibits a higher hemostatic effect. The bulk density is more preferably 0.2 to 3.0 g/cm$^3$, still more preferably 0.25 to 2.5 g/cm$^3$, further preferably 0.3 to 2.0 g/cm$^3$, further more preferably 0.4 to 1.5 g/cm$^3$, particularly preferably 0.5 to 0.9 g/cm$^3$.
The bulk density can be measured as follows.

<Method for measuring bulk density>

Magnesium stearate is applied to a mold and a die (Φ8 mm, angled corner). Subsequently, the mold is filled with the alginate (fill depth 10 mm), followed by tableting using a manual desktop tableting machine. The resulting tablet is weighed. The bulk density is determined by the following equation.

$$\texttt{Bulk density (g/cm}^3\texttt{) = W/V}$$

W: Weight (g) of tablet
V: Inner volume of mold (0.5024 cm$^3$)

Preferably, the alginate passes through a sieve with an aperture of 2000 μm, and 80% or more of the particles of the alginate do not pass through a sieve with an aperture of 10 μm. The percentage of such particles is more preferably 90% or more. The alginate containing particles with 80% or more thereof not passing through a sieve with an aperture of 10 μm can be sufficiently prevented from scattering in the air when it is used as a hemostatic material. The alginate passing through a sieve with an aperture of 2000 μm can easily be sprayed and can facilitate the treatment of a bleeding site.
The alginate can pass through a sieve with an aperture of more preferably 1000 μm, still more preferably 800 μm, further preferably 600 μm, particularly preferably 500 μm.
The alginate cannot pass through a sieve with an aperture of more preferably 20 μm, still more preferably 50 μm, further preferably 80 μm, particularly preferably 100 μm.
The alginate has a degree of swelling of preferably 80% or more, more preferably 100% or more as determined by the following method.

<Method for measuring degree of swelling>

A 0.1 g portion of a sample is weighed into a vial. The height of the sample charged in the vial is measured with a caliper or the like (initial height h0). Next, 2 mL of physiological saline is slowly poured into the vial, and the vial is allowed to stand still at room temperature. After two minutes from the addition of physiological saline, the vial is turned upside down, and then the height (ht) of the swollen sample settled at the bottom of the vial is measured. The degree of swelling is calculated by the following equation.

$$\texttt{Degree of swelling (\%) = (ht - h0)/h0 × 100}$$

h0: Height (mm) of charged sample before swelling
ht: Height (mm) of swollen sample after swelling

The alginate may be in any form, including powder, fiber (cotton-like shape, sheet shape, etc.), gel, solution, and putty. Preferably, the alginate is powder. The alginate in the form of powder can rapidly cover a bleeding site regardless of the part and the shape of the injured area and is likely to stay at the bleeding site. The alginate can more quickly come in contact with moisture in blood and thus have a better hemostatic effect.

<Method for producing alginate>

The alginate of the present invention may be produced by any method including a usual method. The alginate can be produced by subjecting alginic acid to replacement by a monovalent cation and calcium ion, by subjecting a monovalent salt of alginic acid to replacement by calcium ion, or by replacing calcium ion in calcium alginate by a monovalent cation. Preferably, the alginate is produced through a step (A) of mixing a monovalent salt of alginic acid and a calcium salt and a step (B) of desalinating/dehydrating the product obtained in the step (A).

In the step (A) of the method for producing the alginate, the monovalent salt of alginic acid may be any salt, and examples thereof include an alkali metal salt such as a sodium salt or a potassium salt and an ammonium salt. Sodium alginate and potassium alginate are preferred, with sodium alginate being more preferred.

Sodium alginate, potassium alginate, and ammonium alginate are soluble in water. Moreover, they are commercially available and thus can be easily obtained.

The calcium salt used in the step (A) of the method for producing the alginate may be any compound containing a calcium element. Examples thereof include calcium chloride, calcium sulfate, calcium nitrate, calcium carbonate, calcium phosphate, calcium bicarbonate, and calcium hydroxide.

The calcium salt is preferably calcium chloride.

The step (A) of the method for producing the alginate may be any step in which a monovalent salt of alginic acid can be mixed with a calcium salt. The step preferably includes mixing an aqueous sodium alginate solution with an aqueous calcium salt solution.

In the step (A) using an aqueous solution of a monovalent salt of alginic acid, the concentration of the monovalent salt of alginic acid is not limited. The concentration in terms of sodium alginate is preferably 0.1 to 50 wt/v%, more preferably 0.5 to 30 wt/v%, still more preferably 1 to 20 wt/v%.

The concentration of the monovalent salt of alginic acid is a concentration in terms of sodium alginate. In the case where the monovalent salt of alginic acid is potassium alginate, the concentration is calculated while substituting potassium with sodium.

In the step (A) using an aqueous calcium salt solution, the concentration of the calcium salt may be appropriately controlled depending on a target calcium content.

The step (A) preferably includes stirring after mixing a monovalent salt of alginic acid with a calcium salt. The duration of the stirring is not limited. The duration is preferably 1 to 30 minutes, more preferably 5 to 10 minutes.

The step (A) preferably includes a step of adding an organic solvent after mixing a monovalent salt of alginic acid with a calcium salt and subsequent optional stirring to facilitate filtration of the resulting coarse alginate.

Non-limiting examples of the organic solvent include lower alcohols such as methanol, ethanol, and propanol. Ethanol is preferred among these.

The concentration of the ethanol is not limited. The concentration is preferably 30 to 100 vol%, more preferably 50 to 100 vol%.

The step (A) preferably includes a step of filtering the resulting coarse alginate after mixing a monovalent salt of alginic acid with a calcium salt and subsequent optional stirring and optional addition of an organic solvent.

The filtration step is not limited and may be performed by a usual method. The filtration method is preferably filtration through a filter.

Preferably a glass filter is used in the filtration step.

The glass filter has a pore size of preferably 5 to 120 $\mu$m, more preferably 20 to 50 $\mu$m.

The glass filter is preferably a 2G glass filter or a 3G glass filter.

The step (B) of the method for producing the alginate of the present invention includes desalinating/dehydrating the product obtained in the step (A).

The desalinating/dehydrating in the step (B) may be performed by any method, including a usual method such as filtration or dialysis. Filtration is preferred.

The step (B) preferably includes filtration after adding a desalination solvent to the product obtained in the step (A) and subsequent stirring.

Examples of the desalination solvent include water, organic solvents such as methanol, ethanol, and propanol, and mixtures of these solvents. The desalination solvent is preferably water, ethanol, or a solvent mixture of water and ethanol, more preferably 50 to 80 vol% ethanol.

The step (B) of the method for producing the alginate preferably includes a step of drying the product obtained in the step (B).

The drying step may be any step that can evaporate the solvent from the product. The drying step is preferably performed by air drying, drying under reduced pressure, spray drying, or the like.

The drying temperature in the drying step is not limited. The drying temperature is preferably 30°C to 200°C.

The duration of the drying in the drying step is not limited. The duration is preferably 1 to 24 hours.

The method for producing the alginate preferably includes a step of grinding and/or sieving the product obtained in the drying step.

The grinding method is not limited. For example, the griding can be performed using a grinding mill or the like.

The aperture of the sieve used in the sieving is not limited. Preferably, the aperture is 10 to 2000 $\mu$m.

<Hemostatic material>

The alginate of the present invention exhibits a high hemostatic effect and therefore can suitably be used as a hemostatic material.

Herein, the term "hemostatic material" is intended to include pharmaceuticals and medical devices defined in Article 2 of the Pharmaceutical and Medical Device Act. Thus, a hemostatic material in the present invention includes a hemostatic agent.

The present invention also relates to a hemostatic material containing the alginate of the present invention.

The hemostatic material promotes hemostasis and can be used in various applications for local hemostasis in mammals (animals including livestock animals and companion animals and humans). For example, the hemostatic material can be used as a hemostatic material for injured areas, a surgical hemostatic material, or the like and is preferably used as a surgical hemostatic material.

The hemostatic material of the present invention is preferably sprayed to a bleeding affected area during a surgery. The use of the hemostatic material as described above is one aspect of the present invention.

The use of the alginate of the present invention including a step of spraying the alginate to a bleeding affected area in a surgery is also one aspect of the present invention.

The alginate content of the hemostatic material is not limited. The alginate content relative to 100% by mass of the hemostatic material is preferably 50 to 100% by mass. The alginate content is more preferably 60 to 100% by mass, still more preferably 70 to 100% by mass, further preferably 80 to 100% by mass, further more preferably 90 to 100% by mass, particularly preferably 95 to 100% by mass, most preferably 100% by mass.

The hemostatic material may contain another component other than the alginate.

The another component may be any component that does not impair the hemostatic effect and is usable in a living body. Examples of the component include pharmacological components such as an antimicrobial agent, an antibiotics, an anti-inflammatory agent, a blood circulation enhancer, a steroid, an enzyme inhibitor, a growth factor, and various vitamins and additives such as an excipient, a binder, a lubricant, a pH adjuster, a buffer, an antiseptic agent, an antioxidant, a colorant, and a desiccant.

The percentage of the another component in the hemostatic material is not limited. The percentage relative to 100% by mass of the hemostatic material is preferably 0 to 50% by mass, more preferably 0 to 40% by mass, still more preferably 0 to 30% by mass, further preferably 0 to 20% by mass, further more preferably 0 to 10% by mass, particularly preferably 0 to 5% by mass, most preferably 0% by mass.

EXAMPLES

The present invention is described in more detail below with reference to examples. However, the present invention is not limited to these examples.

The physical properties of the alginate are measured as described below.

<Calcium content>

The alginate is pressurized and molded into a disc shape. Then, the intensities of the fluorescent X-rays of a calcium element are measured under the conditions described below using an energy-dispersive X-ray fluorescence spectrometer (Jeol Ltd., JSX-3100RII).

A calibration curve was constructed based on the intensities of fluorescent X-rays of a calcium element in a reference material mixture described below. The calcium content (%) was calculated from the calibration curve and the intensities of the fluorescent X-rays of the calcium element in the alginate.

    Tube voltage: 30 kV
    Collimeter: 7 mm$\phi$
    Atmosphere: vacuum
    Duration of measurement: 120 seconds
    Reference material: a material prepared by mixing sodium alginate (KIMICA, molecular weight: 300000, M/G ratio: 1.1) with calcium alginate (synthesized from the sodium alginate, molecular weight: 300000, M/G ratio: 1.1) at a mass percentage (10, 20, 40, 60, 80, or 90% by mass) to give a homogeneous mixture and then pressure molding the mixture into a disc shape

<Mass average molecular weight>

The alginate was dissolved in a 0.1 w/v% sodium carbonate solution. The resulting solution was subjected to gel filtration chromatography under the following conditions to measure the mass average molecular weight.

    Column: two pieces of TSKgel GMPW$_{XL}$ (Tosoh Corporation, diameter 7.8 mm $\times$ 300 mm)

Column temperature: 40°C
Eluent: 200 mM sodium nitrate solution
Sample concentration: 0.1%
Flow rate: 0.7 mL/min
Amount charged: 50 μL
Detector: RI (differential refractometer)
Reference material: pullulan (molecular weight: 739000, 334000, 216000, 107000, 49700, 22000, 9800, 6300)

<Bulk density>

Magnesium stearate was applied to a mold and a die (φ8 mm, angled corner). Subsequently, the mold was filled with the alginate (fill depth 10 mm) and scraped off.

Tableting was performed at 600 kgf using a manual desktop tableting machine (Ichihashi Seiki, HANDTAB-200). The resulting tablet was weighed. The bulk density was determined by the following equation.

$$\text{Bulk density } (g/cm^3) = W/V$$

W: Weight (g) of tablet
V: Inner volume of mold (0.5024 cm$^3$)

<Degree of swelling>

A 0.1 g portion of a sample was weighed into a vial. The height of the sample charged in the vial was measured with a caliper or the like (initial height h0). Next, 2 mL of physiological saline was slowly poured into the vial, and the vial was allowed to stand still at room temperature. After two minutes or five minutes from the addition of physiological saline, the vial was turned upside down, and then the height (ht) of the swollen sample settled at the bottom of the vial was measured. The degree of swelling was calculated by the following equation.

$$\text{Degree of swelling } (\%) = (ht - h0)/h0 \times 100$$

h0: Height (mm) of charged sample before swelling
ht: Height (mm) of swollen sample after swelling

<Evaluation of hemostasis>

A swine (fasting from the day before, no restriction on water intake) under general anesthesia (induction: isoflurane, concentration of 5%, oxygen flow rate of about 3.0 L/min, maintenance: isoflurane, concentration of 1 to 3%, oxygen flow rate of about 3.0 L/min) was subjected to laparotomy. Subsequently, a biopsy trepan (KAI industries co., ltd., single-use tissue biopsy needle) was inserted into the liver to create an injured area with a width of 4 mm and a depth of 3 mm.

The time taken until the injured area was filled with blood was measured and scored according to "Evaluation criteria for degree of bleeding" in Table 1. Injured areas with Grades 0.5 to 4 were used for the test.

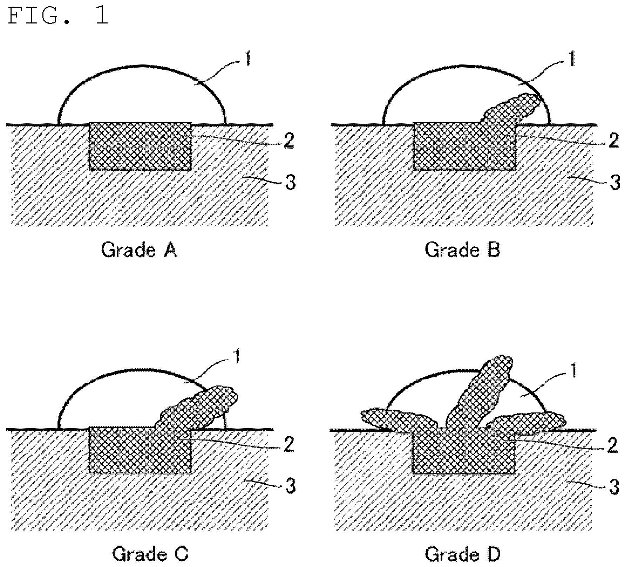

Next, 0.2 g of each test sample was sprayed to the injured area using an Alt shooter (Kaigen Pharma Co., Ltd., mechanical needleless injector for pharmaceuticals and vaccines). After two minutes and five minutes from the spraying, the degree of hemostasis of the injured area was determined according to "Evaluation criteria for the degree of hemostasis" in Table 2. Grade A or B was considered effective. FIG. 1 schematically shows injured areas with Grades A to D as determined according to "Evaluation criteria for degree of hemostasis" in Table 2.

When it was difficult to score, presence or absence of bleeding was judged after the blood was absorbed with gauze.

When the degree of hemostasis after two minutes from the spraying was scored Grade C or D, the subsequent evaluations were not performed.

Injured areas (oozing bleeding) at multiple parts were evaluated for each test substance. The percentage of the determined results with Grade A or B was taken as an efficacy rate.

Further, the blood pressures of the swine were measured during the test. The blood pressures upon creation of the injured areas were recorded for each test substance. A blood pressure lower than 80 mmHg was excluded.

An electrocautery was used for the hemostasis of bleeding sites where hemostasis had not been achieved.

[Table 1]

| Evaluation criteria for degree of bleeding | |
| --- | --- |
| Grade | Degree of bleeding |
| 0 (Exception) | No bleeding |
| 0.5 | 4 seconds or more until blood fills injured area |
| 1 | 3 - 4 seconds |
| 2 | 2 - 3 seconds |
| 3 | 1 - 2 seconds |
| 4 | Less than 1 second |
| 5 (Exception) | Spurting bleeding |

[Table 2]

| Evaluation criteria for degree of hemostasis | |
| --- | --- |
| Grade | Degree of hemostasis |
| A | Oozing blood was not present on test substance, and bleeding was not observed. |
| B | Oozing blood was present on test substance, but continuous bleeding was not observed. |
| C | Bleeding (increasing oozing blood) was observed on test substance. |
| D | Bleeding was observed on test substance and at a margin of test substance or at a margin of test substance. |

<Example 1>

An aqueous calcium chloride solution prepared by dissolving 920 mg of calcium chloride dihydrate in 500 mL of water was added to 1 L of a 1.5 w/v% aqueous solution of sodium alginate (KIMICA) and stirred for seven minutes at room temperature. Thereafter, 3 L of anhydrous ethanol was added and stirred for three minutes, and the mixture was allowed to stand still for 30 minutes.

The resulting coarse alginate gel was subjected to suction filtration using a 2G glass filter.

Next, 1.5 L of 70% ethanol was added to the filtered coarse alginate, and they were stirred for three minutes, followed by suction filtration. This desalination operation was repeated one more time. Then, the resulting product was dried in the air at room temperature overnight and subsequently dried under reduced pressure at 40°C for five hours. The alginate thus obtained was ground. The particles of the alginate passing through a sieve with an aperture of 250 $\mu$m were further subjected to sieving using a sieve with an aperture of 150 $\mu$m. The particles that did not pass through the sieves were collected, whereby alginate 1 was obtained.

The various physical properties and hemostatic ability of the alginate 1 were determined. Table 3 shows the results.

<Examples 2 to 10>

Alginates 2 to 10 were prepared as in Example 1, except that sodium alginates (KIMICA) with various molecular weights and M/G ratios were used to change the amount of the calcium chloride to give the calcium contents indicated in Table 3.

The various physical properties and hemostatic ability of the alginates 2 to 10 were determined. Table 3 shows the results.

<Comparative Examples 1 to 5>

Sodium alginate (KIMICA), cellulose (Sigma-Aldrich), microporous starch (Medicon, Bard® Arista® AH), or oxidized regenerated cellulose (Johnson & Johnson, Surgicel® powder absorbable hemostat) was used in Comparative Examples 1 to 5. The various physical properties and hemostatic ability of these materials were determined. Table 3 shows the results. The cellulose was evaluated twice, specifically, in Comparative Examples 2 and 5. In the evaluation method for Comparative Example 4, when the vial was turned upside down after two minutes and five minutes from the addition of physiological saline, the sample was not settled at the bottom of the vial due to the dissolution of the sample, or the like. Thus, the height (ht) of the swollen sample after swelling was zero and therefore could not be evaluated by the test method.

<Pathological examination of bleeding site (injured area)>

In the hemostasis evaluation in Example 9, alginate 9 was sprayed to a bleeding site. After confirming that hemostasis was achieved after five minutes from the spraying, an appropriate size of a portion including the site was cut out of the liver.

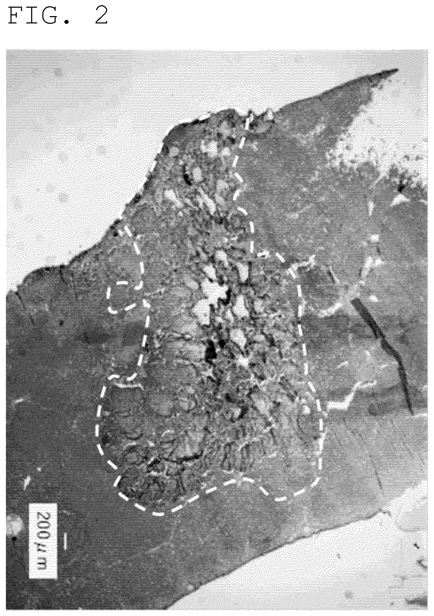

The isolated liver portion was fixed with a Bouin's solution (Fujifilm Wako Pure Chemicals Co., Ltd.) and was then thinly sliced by a usual method. The resulting piece was stained with hematoxylin-eosin and observed by a microscope. FIG. 2 shows the observed micrograph.

In FIG. 2, the part surrounded by the dashed line corresponds to the alginate. Presumably, the alginate came into contact with moisture in blood to block the bleeding site, thereby exhibiting a local hemostatic effect.

[Table 3]

| | Test substance | Ca content (%) | Molecular weight | M/G ratio | Bulk density (g/cm³) | Degree of swelling (%) | | Degree of bleeding (Median value) | Efficiency of hemostasis | |
|---|---|---|---|---|---|---|---|---|---|---|
| | | | | | | After two minutes | After five minutes | | After two minutes from application | After five minutes from application |
| Example 1 | Alginate 1 | 27.1 | 357,906 | 1.1 | 0.68 | 115 | 103 | 2 | 76% (16/21 cases) | 71% (15/21 cases) |
| Example 2 | Alginate 2 | 50.7 | 358,928 | 1.1 | 0.49 | 262 | 250 | 2 | 95% (20/21 cases) | 86% (18/21 cases) |
| Example 3 | Alginate 3 | 74.1 | 364,118 | 1.1 | 0.55 | 372 | 384 | 3 | 100% (21/21 cases) | 95% (20/21 cases) |
| Example 4 | Alginate 4 | 81.4 | 50,404 | 0.9 | 0.75 | 76 | 80 | 3 | 70% (14/20 cases) | 70% (14/20 cases) |
| Example 5 | Alginate 5 | 79.3 | 333,216 | 1.3 | 0.71 | 193 | 133 | 3 | 95% (19/20 cases) | 95% (19/20 cases) |
| Example 6 | Alginate 6 | 83.6 | 1,528,719 | 1.4 | 0.57 | 393 | 522 | 3 | 90% (18/20 cases) | 85% (17/20 cases) |
| Example 7 | Alginate 7 | 83.7 | 303,209 | 1.3 | 0.61 | 176 | 170 | 3 | 80% (16/20 cases) | 80% (16/20 cases) |
| Example 8 | Alginate 8 | 43.2 | 311,745 | 1.3 | 0.61 | 190 | 171 | 3.5 | 75% (15/20 cases) | 75% (15/20 cases) |
| Example 9 | Alginate 9 | 82.0 | 330,637 | 1.9 | 0.86 | 352 | 345 | 3 | 75% (15/20 cases) | 75% (15/20 cases) |
| Example 10 | Alginate 10 | 79.9 | 304,184 | 1.1 | 0.58 | 306 | 285 | 3 | 90% (27/30 cases) | 87% (26/30 cases) |
| Comparative Example 1 | Sodium alginate | 0 | 379,246 | 1.1 | 0.50 | 129 | 143 | 3 | 38% (8/21 cases) | 33% (7/21 cases) |
| Comparative Example 2 | Cellulose | - | - | - | 0.40 | 33 | 52 | 2.5 | 33% (2/6 cases) | 16% (1/6 cases) |
| Comparative Example 3 | Microporous starch | - | - | - | 0.51 | 68 | 67 | 3 | 0% (0/30 cases) | 0% (0/30 cases) |
| Comparative Example 4 | Oxidized regenerated cellulose | - | - | - | 0.55 | - | - | 3 | 50% (15/30 cases) | 43% (13/30 cases) |
| Comparative Example 5 | Cellulose | - | - | - | 0.40 | 33 | 52 | 3 | 20% (2/10 cases) | 0% (0/10 cases) |

The results in Table 3 demonstrate that the alginate of the present invention has a better hemostatic effect than sodium alginate and cellulose.

REFERENCE SIGNS LIST

1  test substance

2  blood

3  liver

**Claims**

1. An alginate, comprising

   a calcium salt of a carboxyl group and
   a different carboxyl (salt) group,
   wherein, provided that a structural unit containing the calcium salt in a repeating unit (structural unit) constituting the alginate is calcium alginate, a structural unit containing the different carboxyl (salt) group in the repeating unit is sodium alginate, and the alginate is regarded as a mixture of the sodium alginate and the calcium alginate, the alginate has a mass percentage of the calcium alginate (calcium content) of 1 to 99% by mass.

2. The alginate according to claim 1,
   wherein the alginate has a mass average molecular weight of 10000 or more.

3. The alginate according to claim 1 or 2,

   wherein the alginate comprises a mannuronate-derived structural unit and a gluronate-derived structural unit, and
   when all cations in the alginate are replaced by protons, the alginate has a ratio of a mannuronic acid-derived structural unit to a gluronic acid-derived structural unit (mannuronic acid/gluronic acid) of 0.6 or more.

4. The alginate according to any one of claims 1 to 3,
   wherein the alginate has a bulk density of 0.2 g/cm$^3$ or more.

5. A hemostatic material comprising the alginate according to any one of claims 1 to 4.

FIG. 1

Grade A

Grade B

Grade C

Grade D

FIG. 2

## INTERNATIONAL SEARCH REPORT

International application No.

**PCT/JP2023/021937**

### A. CLASSIFICATION OF SUBJECT MATTER

***C08B 37/04***(2006.01)i; ***A61K 31/734***(2006.01)i; ***A61L 31/04***(2006.01)i; ***A61P 7/04***(2006.01)i
FI:  C08B37/04; A61L31/04 120; A61P7/04; A61K31/734

According to International Patent Classification (IPC) or to both national classification and IPC

### B. FIELDS SEARCHED

Minimum documentation searched (classification system followed by classification symbols)

C08B37/04; A61K31/734; A61L31/04; A61P7/04

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN), JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X | JP 04-501067 A (BRITCAIR LIMITED) 27 February 1992 (1992-02-27) processes 1-4, treatments 5, 6, examples 1-6, claims | 1-5 |
| X | GB 1328088 A (WALLACE, CAMERON AND CO., LTD.) 30 August 1973 (1973-08-30) examples 1-4, claims | 1-5 |
| X | CN 106267300 A (BEIJING UNIVERSITY OF CHEMICAL TECHNOLOGY, PEOP. REP. CHINA; INSTITUTE OF PHARMACOLOGY AND TOXICOLOGY ACADEMY OF MILITARY MEDICAL SCIENCES PLA) 04 January 2017 (2017-01-04) example 3, paragraphs [0048]-[0054], claims 3, 5 | 1-5 |
| X | JP 48-026883 A (MEDICAL ALGINATES LIMITED) 09 April 1973 (1973-04-09) p. 5, lower left column, claims | 1-5 |
| X | GB 1394741 A (MEDICAL ALGINATES LIMITED) 21 May 1975 (1975-05-21) p. 1, left column, lines 13-33, examples 1-12 | 1-5 |

✓ Further documents are listed in the continuation of Box C.   ✓ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **23 August 2023** | **05 September 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

International application No.

**PCT/JP2023/021937**

| C. | DOCUMENTS CONSIDERED TO BE RELEVANT | |
|---|---|---|
| Category* | Citation of document, with indication, where appropriate, of the relevant passages | Relevant to claim No. |
| X | SEGAL, Helen C. et al. The effects of alginate and non-alginate wound dressings on blood coagulation and platelet activation. Journal of Biomaterials Applications. 1998, vol. 12(3), pp. 249-257<br>tables 1, 2, 3, 4 | 1-5 |
| X | JP 2007-504918 A (MARINE POLYMER TECHNOLOGIES, INC.) 08 March 2007 (2007-03-08)<br>claim 30 | 1-5 |

Form PCT/ISA/210 (second sheet) (January 2015)

INTERNATIONAL SEARCH REPORT
Information on patent family members

International application No.

**PCT/JP2023/021937**

| Patent document cited in search report | | | Publication date (day/month/year) | Patent family member(s) | | | Publication date (day/month/year) |
|---|---|---|---|---|---|---|---|
| JP | 04-501067 | A | 27 February 1992 | US example WO EP | 5238685 1990/001954 0433354 | A A1 A1 | |
| GB | 1328088 | A | 30 August 1973 | (Family: none) | | | |
| CN | 106267300 | A | 04 January 2017 | (Family: none) | | | |
| JP | 48-026883 | A | 09 April 1973 | US column 6 US GB DE | 3824997 3879168 1375572 2209383 | A A A A1 | |
| GB | 1394741 | A | 21 May 1975 | (Family: none) | | | |
| JP | 2007-504918 | A | 08 March 2007 | WO claim 30 US US EP | 2005/027993 2012/0220958 2005/0075597 1673134 | A2 A1 A1 A2 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013077414 A

- JP 2008515927 T

**Non-patent literature cited in the description**

- *Carbohydrate Research*, 1974, vol. 32, 217-225